# EUROPEAN PATENT APPLICATION

(11) **EP 2 011 857 A1**
(43) Date of publication of application: **07.01.2009**
(21) Application number: 07742238.4
(22) Date of filing: 24.04.2007
(51) Int. Cl.: C12M 3/00

(54) **METHOD OF PRODUCING CELL CULTURE CONTAINER**

(30) Priority: 26.04.2006 JP 2006122617
(71) Applicant: Toyo Gosei Co., Ltd., Ichikawa-shi, Chiba 272-0012 (JP)
(72) Inventor: IKEYA, Takeshi, Inba-gun, Chiba 270-1609 (JP); WATANABE, Masaharu, Inba-gun, Chiba 270-1609 (JP); MIYAZAKI, Kana, Inba-gun, Chiba 270-1609 (JP); SHIBUYA, Toru, Inba-gun, Chiba 270-1609 (JP)
(74) Representative: Strehl Schübel-Hopf & Partner
(86) International application number: PCT/JP2007/058803
(87) International publication number: WO 2007/125894

(57) **Abstract**

To produce a cell culture container which is suitable for use in, for example, biochemical experiments, clinical experiments, and research and development of drugs. The production of the cell culture container includes providing a cell culture container base 1 having a plurality of wells serving as regions for culturing cells; applying a hydrophilic photosensitive composition to the bottom surface of each of the wells, to thereby form a coating 3; subjecting the coating 3 to patternwise light exposure; and removing an uncured portion of the coating through development, to thereby yield a cell culture container having, on the bottom surface of each well, a patterned hydrophilic coating layer formed of a photo-cured product.

## Description

### Technical Field

The present invention relates to a method for producing a cell culture container which is suitable for use in, for example, biochemical experiments, clinical experiments, and research and development of drugs.

### Background Art

A variety of cell culture containers have been employed in biochemical experiments, clinical experiments, and research and development of drugs. For example, many attempts have been made to form a layer for preventing adhesion of cells thereto (hereinafter the layer may be referred to as a "cell-adhering-prevention layer") or a patterned cell-adhering-prevention layer on a glass slide, a coverslip, or a plastic plate. There has been proposed a cell culture container including a plate substrate having thereon, as a cell-adhering-prevention layer, a patterned layer formed of a photo-cured product of a photosensitive composition predominantly containing a water-soluble polymer (see Patent Document 1). Since the technique disclosed in Patent Document 1 employs a plate substrate, a patterned layer can be readily formed on the substrate with high accuracy and at high productivity by, for example, applying the photosensitive composition to the substrate through spin coating.

However, in general, when a plate substrate having a patterned layer on a surface thereof is employed for culturing cells in, for example, biochemical experiments, clinical experiments, or research and development of drugs, an additional process is required. One process includes bonding a plate substrate to the surface of a dish (i.e., a culture container) and subsequently adding an aqueous solution containing cells to the dish, and another process includes placing a plate substrate in a culture dish containing an aqueous solution containing cells. The former process poses problems in that an intricate operation is required for bonding of the substrate to the dish, and that a compound eluted from an employed adhesive may adversely affect the cells, whereas the latter process poses a problem in that cells are deposited not only on the patterned-layer-formed surface (top surface) of the substrate immersed in the dish, but also on the bottom surface of the substrate.

Patent Document 1: Japanese Patent Application Laid-Open (*kokai*) No. 2005-280076 (Claim 9, Paragraph numbers [0093] and [0094], etc.)

### Disclosure of the Invention

### Problems to be Solved by the Invention

In view of the foregoing, an object of the present invention is to provide a method for producing a cell culture container which is suitable for use in, for example, biochemical experiments, clinical experiments, and research and development of drugs.

### Means for Solving the Problems

Accordingly, in a first mode of the present invention for attaining the aforementioned object, there is provided a method for producing a cell culture container, characterized by comprising providing a cell culture container base having a plurality of wells serving as regions for culturing cells; applying a hydrophilic photosensitive composition to the bottom surface of each of the wells, to thereby form a coating; subjecting the coating to patternwise exposure to light (hereinafter may be referred to simply as "patternwise light exposure"); and removing an uncured portion of the coating through development, to thereby yield a cell culture container having, on the bottom surface of each well, a patterned hydrophilic coating layer formed of a photo-cured product of the composition.

A second mode of the present invention is drawn to a specific embodiment of the cell culture container production method according to the first mode, wherein the hydrophilic photosensitive composition contains a photosensitive resin having a water-soluble polymer backbone and a photo-crosslinkable photosensitive group.

A third mode of the present invention is drawn to a specific embodiment of the cell culture container production method according to the first or second mode, wherein the patternwise light exposure is carried out using a mask having protrusions, each protrusion having a pattern of interest on the tip end thereof which faces the bottom surface of a corresponding well of the cell culture container and being inserted in the well such that the tip end of the protrusion comes into close contact with or is disposed proximately to the coating formed on the bottom surface of the well.

### Effects of the Invention

The method for producing a cell culture container of the present invention can readily produce a cell culture container having a plurality of wells serving as regions for culturing cells, each of the wells having, on the bottom surface thereof, a patterned cell-adhering-prevention layer. The thus-produced cell culture container is suitable for use in, for example, biochemical experiments, clinical experiments, and research and development of drugs.

### Brief Description of the Drawings

[Fig. 1]

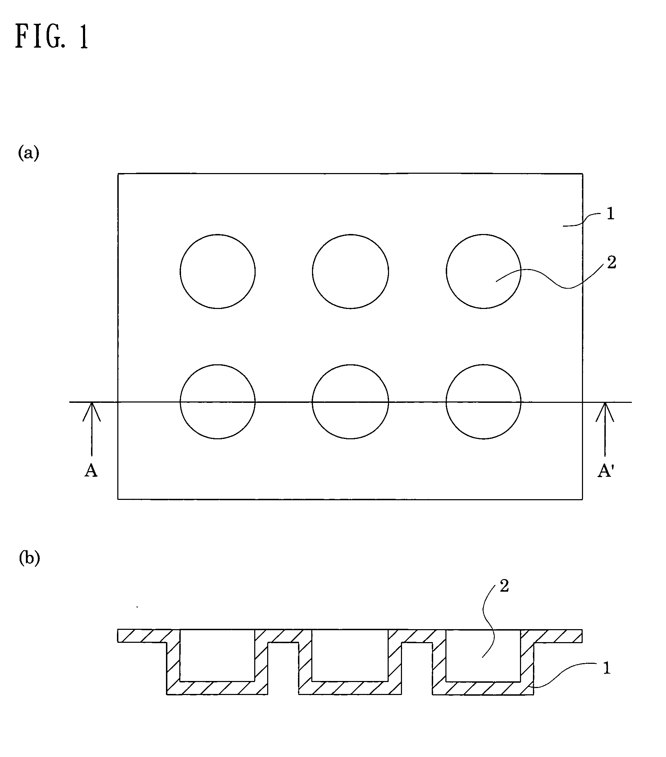

   Fig. 1 shows a step of the cell culture container production method of the present invention.
[Fig. 2]

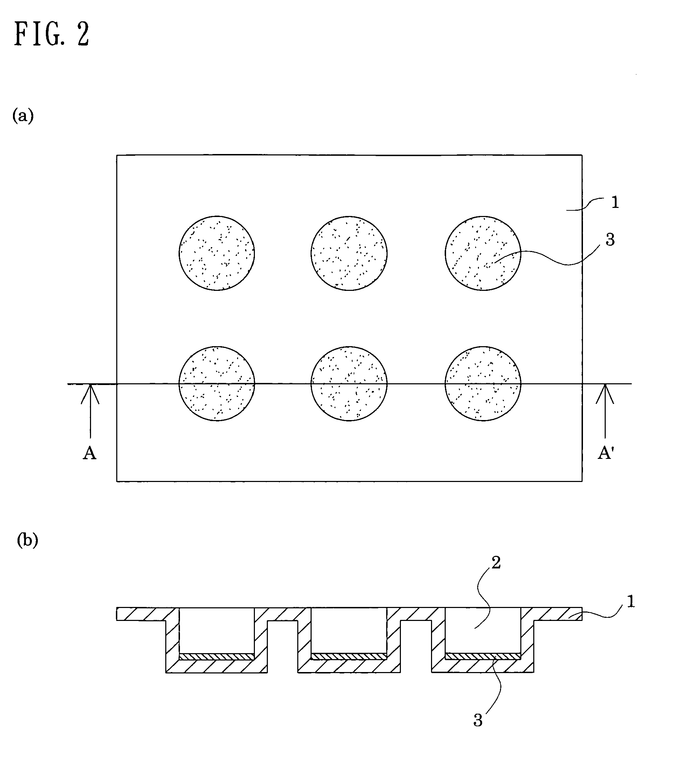

   Fig. 2 shows another step of the cell culture container production method of the present invention.
[Fig. 3]

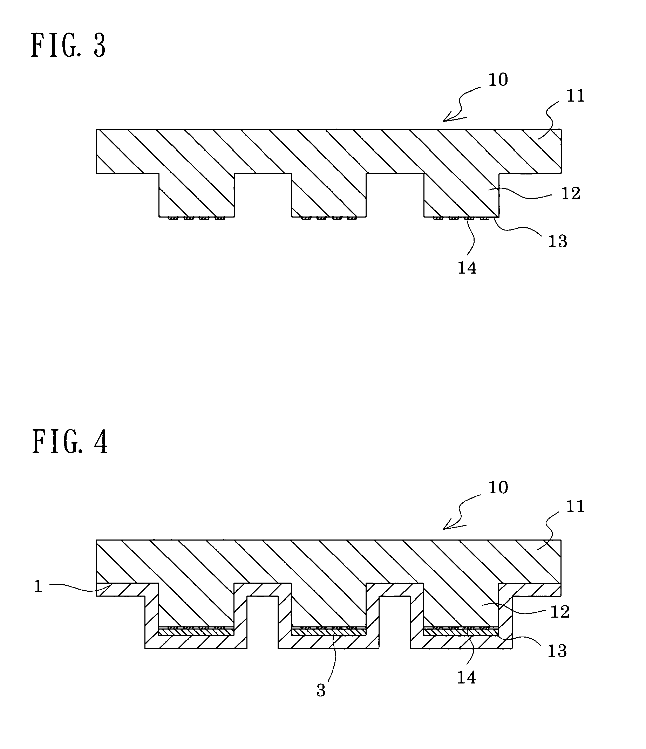

   Fig. 3 shows a specific example of a mask.
[Fig. 4]
   Fig. 4 shows still another step of the cell culture container production method of the present invention.
[Fig. 5]

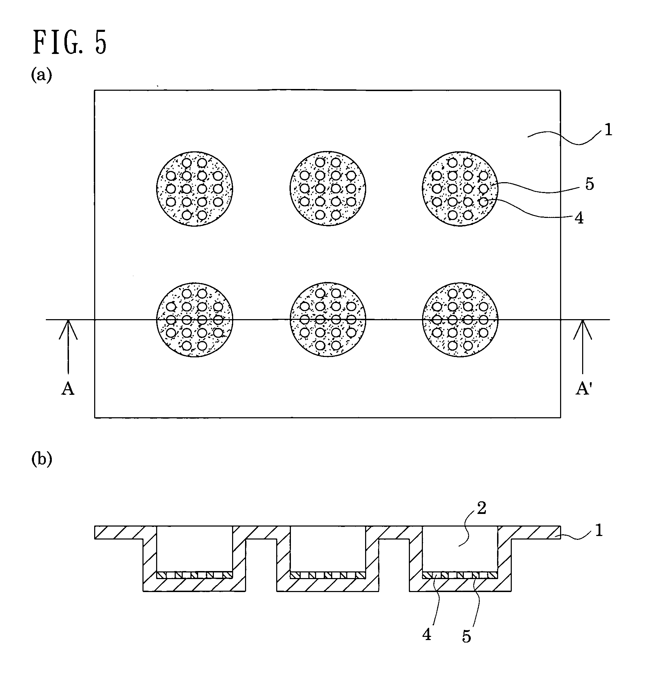

   Fig. 5 shows yet another step of the cell culture container production method of the present invention.
[Fig. 6]

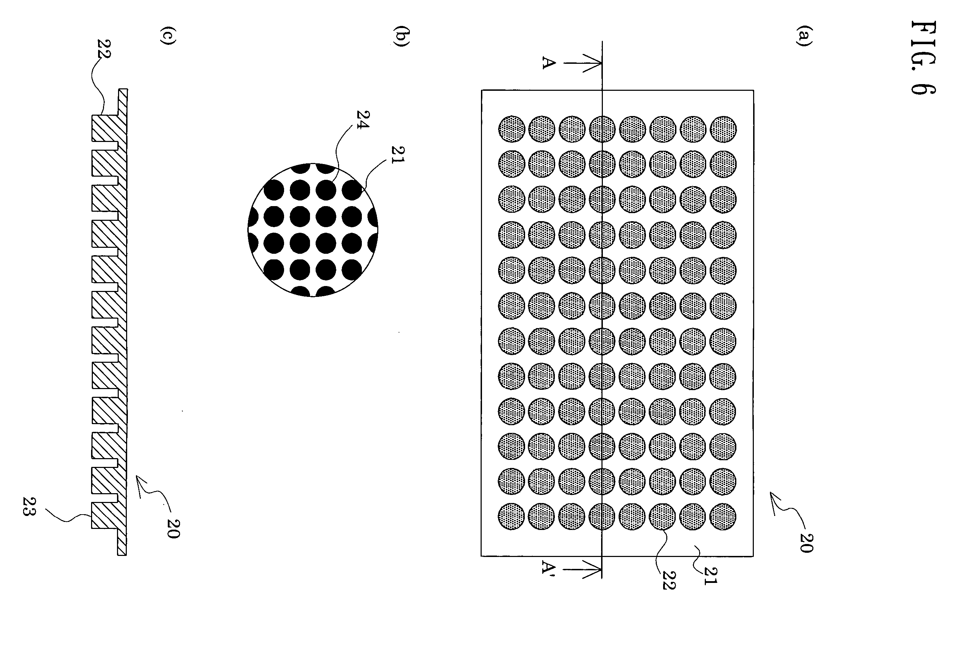

   Fig. 6 schematically shows a mask employed in Example 11.
[Fig. 7]

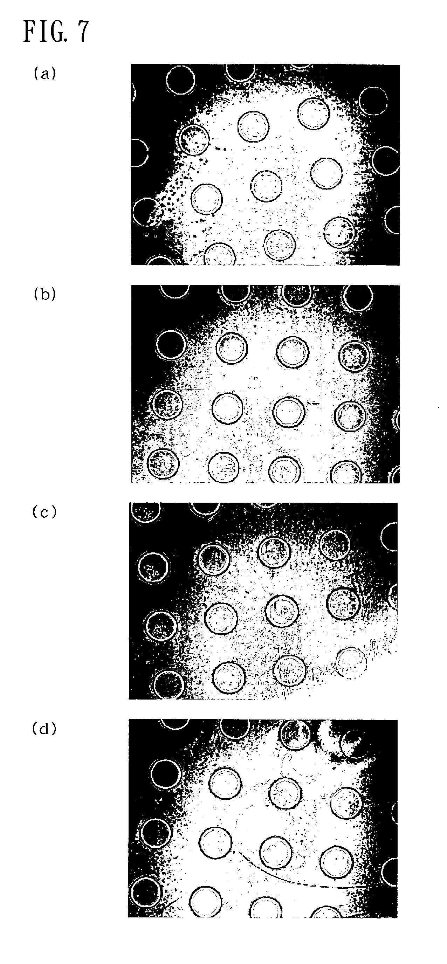

   Fig. 7 shows the results of Test Example.

### Description of Reference Numerals

- 1:: Cell culture container base
- 2:: Well
- 3:: Coating
- 4:: Hole
- 5:: Pattern
- 10,: 20: Mask
- 11:: Mask base
- 12:: Protrusion
- 13:: Tip end
- 14:: Pattern

### Best Modes for Carrying Out the Invention

The present invention will next be described in detail.

In the method for producing a cell culture container of the present invention, a cell culture container base having a plurality of wells (i.e., regions for culturing cells) is provided; a hydrophilic photosensitive composition is applied to the bottom surface of each of the wells, to thereby form a coating; the coating is subjected to patternwise light exposure; and uncured portions of the coating are removed through development, to thereby yield a cell culture container having, on the bottom surface of each well, a patterned hydrophilic coating layer formed of a photo-cured product of the photosensitive composition.

No particular limitation is imposed on the cell culture container base, so long as it has a plurality of wells. Examples of the cell culture container base which may be employed include multi-well plates which have widely been used in the art such as 6-well, 12-well, 48-well, 96-well, and 384-well plates. Specific examples include a commercially available multi-well plate (width: 75 mm, length: 115 mm) having 96 wells, each having a diameter of 7 mm and a depth of about 12 mm.

No particular limitation is imposed on the material of the cell culture container base. However, from the viewpoint of observing behavior of cells during culturing, preferably, at least the bottom surfaces of wells are colorless and transparent, or virtually colorless and transparent. From this viewpoint, the material of the cell culture container base is preferably, for example, plastic or glass material, particularly preferably plastic material such as vinyl chloride plastics, polystyrene, polypropylene, or acrylic polymer. Since a light exposure process is employed for forming a hydrophilic coating layer on the bottom surfaces of the wells of the cell culture container base, from the viewpoint of suppression of light scattering, etc. from the side surfaces of the wells, the well side surfaces are preferably colored.

The cell culture container base employed in the present invention may have a modified surface. Surface modification may be carried out through any known technique. Such a surface-modified cell culture container base may be, for example, a polystyrene container base having a surface imparted with hydrophilicity through plasma treatment, or a plate coated with a bioactive substance such as poly-L-lysine, laminin, fibronectin, or collagen.

The hydrophilic photosensitive composition applied to the wells of the cell culture container base preferably contains a hydrophilic photosensitive resin. No particular limitation is imposed on the hydrophilic photosensitive resin, so long as a photo-cured product thereof can exhibit an effect of preventing adhesion of cells. However, from the viewpoint of exhibition of such a cell-adhering-prevention effect, the hydrophilic photosensitive resin preferably has a water-soluble polymer serving as a backbone chain, and a photo-crosslinkable photosensitive group introduced to the water-soluble polymer as a side chain or a backbone end group. When a hydrophilic photosensitive composition containing a photosensitive compound of low molecular weight (e.g., a photo-crosslinking agent of low molecular weight) is employed for forming a coating layer, a portion of the low-molecular-weight photosensitive compound remaining unreacted may be eluted from the coating layer during cell culture performed after formation of the layer, resulting in inhibition of natural cell behavior. However, a photosensitive compound of low molecular weight may be employed, so long as, in consideration of adverse effects of the photosensitive compound remaining unreacted, a cell culture system which is not affected by the remaining photosensitive compound can be established, or the amount of remaining photosensitive compound can be controlled to zero.

The water-soluble polymer serving as a backbone chain of the hydrophilic photosensitive resin may be, for example, polyethylene glycol or polyvinyl alcohol. The photo-crosslinkable photosensitive group introduced to the water-soluble polymer preferably has an azido group. Examples of the photosensitive group having an azido group include an azidobenzoic acid group. The hydrophilic photosensitive resin employed may be prepared by, for example, introducing such a photosensitive group to both ends of polyethylene glycol. From the viewpoint of, for example, easy introduction to the water-soluble polymer, the photo-crosslinkable photosensitive group introduced thereto is preferably a photosensitive group represented by the following formula (1):

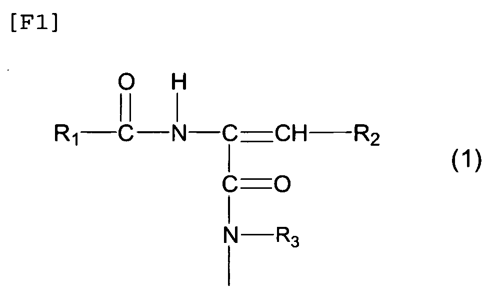

(wherein R₁ is a group selected from among the following formula group (2), and R₂ is a group selected from among the following formula group (3). At least one of R₁ and R₂ has at least one azido group. R₃ represents a hydrogen atom, an alkyl group, an acetal-group-containing alkyl group, an aryl group, an aralkyl group, or a substituent containing a base-forming nitrogen atom, preferably a hydrogen atom, a C1-C6 alkyl group, an acetal-group-containing alkyl group, an aryl group, an aralkyl group, or a substituent containing a base-forming nitrogen atom.)

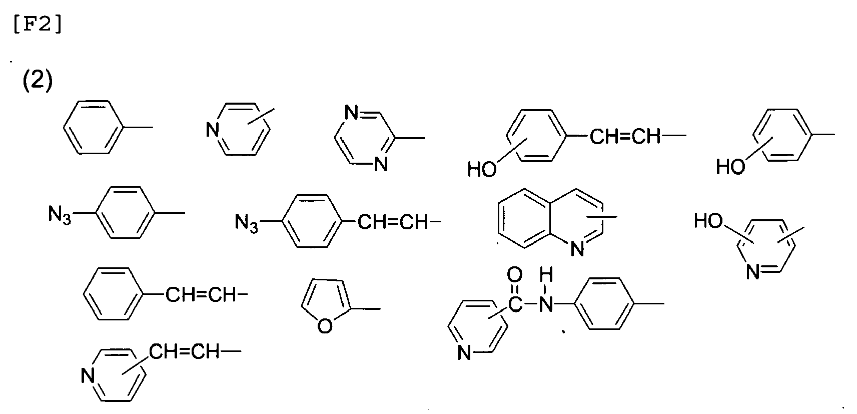

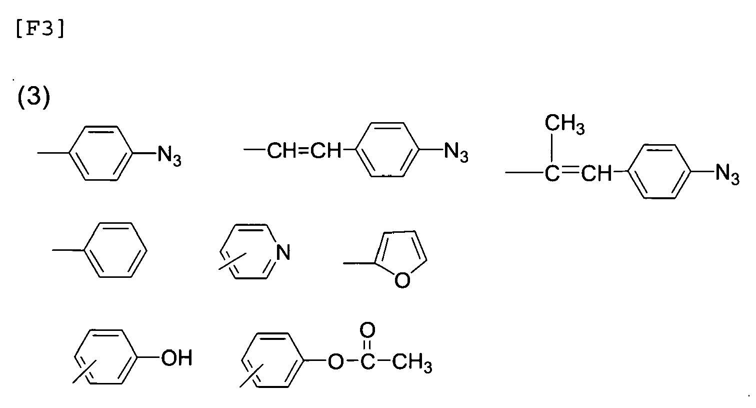

Specific examples of the group represented by formula (1) include structures (1)-1 to (1)-15 shown in Table 1. These structures are represented by formula (1) in which substituents R₁ to R₃ are represented by those listed in Table 1. For example, structures (1)-1 to (1)-4 each have an azido group as R₂, and structure (1)-5 has azido groups as R₁ and R₂. Particularly preferably, R₁ is represented by the following formula (4), and R₂ is represented by the following formula (5).

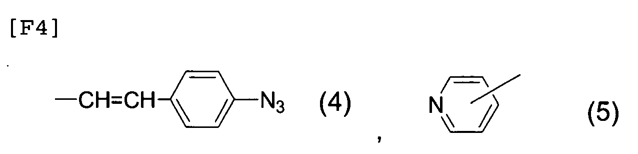

**[Table 1]**

| | R₁ | R₂ | R₃ |
|---|---|---|---|
| (1) -1 | 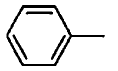 | 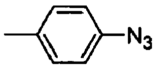 | H |
| (1) -2 | 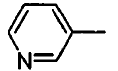 | 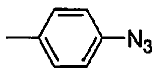 | H |
| (1) -3 | 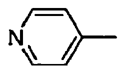 | 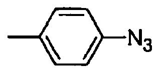 | H |
| (1) -4 | 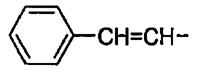 | 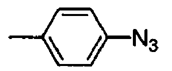 | H |
| (1) -5 | 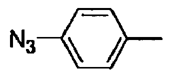 | 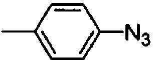 | H |
| (1) -6 | 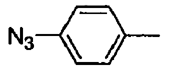 | 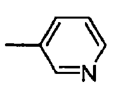 | H |
| (1) -7 | 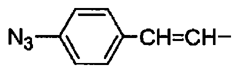 | 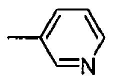 | H |
| (1) -8 | 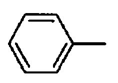 | 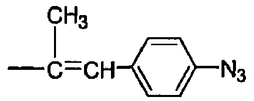 | H |
| (1) -9 | 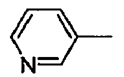 | 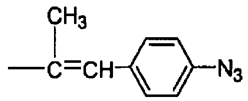 | H |
| (1) -10 | 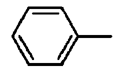 | 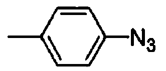 | 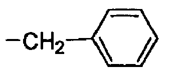 |
| (1) -11 | 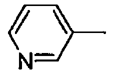 | 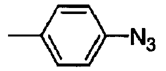 | 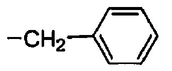 |
| (1) -12 | 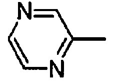 | 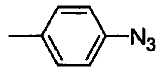 | 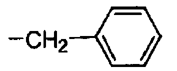 |
| (1) -13 | 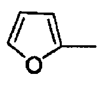 | 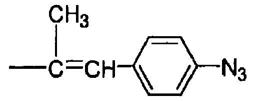 | H |
| (1) -14 | 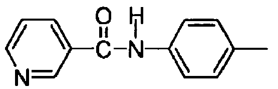 | 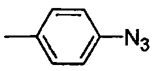 | H |
| (1) -15 | 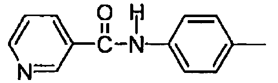 | 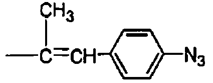 | H |

No particular limitation is imposed on the method for producing such a hydrophilic photosensitive resin, and the resin may be produced through any known method. For example, a hydrophilic photosensitive resin, which is a water-soluble polymer to which a photosensitive group represented by formula (1) has been introduced may be produced by reacting a water-soluble polymer having a side-chain amino group or an end amino group with a compound which forms a structure represented by formula (1) through linking to the amino group. Alternatively, such a hydrophilic photosensitive resin may be produced through the following procedure: a compound having both an acetal group and an amino group is reacted with a compound which forms a structure represented by formula (1) through linking to the amino group, and then the acetal group is reacted with a hydroxyl group of a water-soluble polymer.

Examples of the compound which forms a structure represented by formula (1) through bonding with an amino group of a water-soluble polymer or an acetal compound include photosensitive units disclosed in the published document (Japanese Patent Application Laid-Open (*kokai*) No. 2003-292477), such as 4-((4-azidophenyl)methylene)-2-phenyl-1,3-oxazolin-5-one (photo-functional compound 1), 4-((4-azidophenyl)methylene-2-(3-pyridyl)-1,3-oxazolin-5-one) (photo-functional compound 2), 2-(4-azidophenyl)-4-(3-pyridylmethylene)-1,3-oxazolin-5-one (photo-functional compound 3), 2-(2-(4-azidophenyl)vinyl)-4-(3-pyridylmethylene)-1,3-oxazolin-5-one (photo-functional compound 4), 4-(4-azido-β-methyl-cinnamylidene)-2-phenyl-2-oxazolin-5-one (photo-functional compound 5), and 4-(4-azido-β-methyl-cinnamylidene)-2-(3-pyridyl)-2-oxazolin-5-one (photo-functional compound 6). These photo-functional compounds may be produced through a method disclosed in the published document.

The hydrophilic photosensitive composition is generally a solution produced by dissolving any of the aforementioned hydrophilic photosensitive resins in a solvent. No particular limitation is imposed on the type of the solvent employed, so long as the solvent can dissolve the hydrophilic photosensitive resin, and causes no damage to the cell culture container base. The solvent is preferably water, an organic solvent compatible with water, or a mixture thereof. Examples of the organic solvent compatible with water include alcohols (e.g., ethanol) and dimethyl sulfoxide. Of the aforementioned solvents, water is particularly preferred. This is because, when an organic solvent is employed, the solvent may adversely affect the cell culture container base, or the solvent remaining after photo-curing may adversely affect cells.

The hydrophilic photosensitive composition may contain an additive, so long as the additive does not inhibit formation of a photo-cured product. Examples of the additive include pH regulators for the photosensitive composition; i.e., acids such as mineral acids and organic acids, and bases such as sodium hydroxide, potassium hydroxide, and aqueous ammonia. Also, a salt for regulating salt (ionic) strength such as sodium chloride, a buffer for stabilizing pH such as phosphate buffer, or a defoaming agent may be added to the composition.

When the hydrophilic photosensitive composition is exposed to light, a hydrophilic photo-cured product is produced through photo-crosslinking reaction. In the present invention, the hydrophilic photosensitive composition is applied to the bottom surfaces of wells of the cell culture container base, to thereby form a coating on each of the wells; the coating is subjected to patternwise light exposure; and an uncured portion (i.e., an unexposed portion) of the coating is removed through development, to thereby yield a cell culture container in which each of the wells has, on the bottom surface thereof, a patterned hydrophilic coating layer formed of a photo-cured product of the photosensitive composition.

No particular limitation is imposed on the method for applying the hydrophilic photosensitive composition to the bottom surfaces of wells of the cell culture container base, so long as the composition can be substantially uniformly applied on the well bottom surfaces. For example, preferably, application of the hydrophilic photosensitive composition is carried out through dispensing of a predetermined amount of the composition with a pipette, or by means of a constant-volume dispenser. The amount of the photosensitive composition applied to the cell culture container base may be appropriately determined on the basis of the volume of each well of the container base. When, for example, the photosensitive composition is applied to a commercial multi-well plate having 96 wells, each having a diameter of about 7 mm and a depth of about 12 mm, the amount of the applied photosensitive composition is preferably 5 to 200 µL, particularly preferably 5 to 50 µL. A coating formed from the hydrophilic photosensitive composition preferably has a uniform thickness. The thickness of the coating is preferably about 5 nm to about 10 µm. This is because, a coating having a thickness of less than 5 nm encounters difficulty in determining whether or not the coating has a uniform thickness, whereas a coating having a thickness of more than 10 µm requires a high-viscosity solution of the photosensitive composition, application of which readily causes a problem.

After formation of a coating through application of the photosensitive composition, if necessary, the coating may be thermally treated before exposure to light. No particular limitation is imposed on the thermal treatment conditions, so long as thermal treatment is carried out under such conditions that do not adversely affect the cell culture container or the hydrophilic photosensitive composition. Thermal treatment is generally performed at 4 to 70°C for about 1 minute to about 24 hours, preferably at 20 to 40°C for about 5 minutes to about 1 hour.

A coating formed from the hydrophilic photosensitive composition may be subjected to patternwise light exposure through conventionally known means. Generally, the patternwise light exposure is carried out using a mask having a pattern of interest. The mask employed for producing a photo-cured product having a pattern of interest is preferably a mask having a light-transmitting portion corresponding to the pattern of interest of the photo-cured product, and a light-non-transmitting portion (i.e., a portion other than the pattern-corresponding portion). For example, the mask employed may be an emulsion mask or a chromium mask prepared by depositing an emulsion or chromium, in a pattern of interest, on a mask base made of a material (e.g., glass, quartz, or polymethyl methacrylate) which allows the exposure light to be transmitted through it.

There may be employed a mask having a pattern and a size smaller than the area of the bottom surface of each well of the cell culture container base. When such a mask is employed, masks are individually provided on a coating formed on the wells. Alternatively, there may be employed a mask having a size greater than the area of the bottom surfaces of wells of the cell culture container base, and having protrusions to be inserted into the wells, each protrusion having a pattern of interest on the tip end thereof that faces the bottom surface of a corresponding well of the container. When such a mask is employed, the mask is provided on the cell culture container base so that the tip ends of the protrusions are inserted into the wells, and each of the tip ends comes into close contact with or is disposed proximately to a coating formed on the bottom surface of a corresponding well of the container. From the viewpoint of productivity, a mask having a size greater than the area of the bottom surfaces of wells is preferably employed.

When the coating is subjected to patternwise light exposure by means of the aforementioned masks, preferably, the mask is provided so as to come into close contact with the coating; the mask is provided so as to be disposed proximately to the coating via a liquid layer inactive to the coating; or the mask is provided so as to be disposed proximately to the coating via a gas layer. This is because, when a mask is provided so as to come into close contact with or to be disposed proximately to the coating, due to suppression of interference of light to which the coating is exposed, only a desired specific portion of the coating can be subjected to patternwise light exposure, and a particularly excellent patterned and cured hydrophilic coating layer can be formed after development. When a mask is provided in such a manner, the coating is exposed, through the mask, to light on the side of the mask opposite the side facing the coating. When the coating is formed on a surface of a transparent cell culture container base, a mask having a pattern may be provided so as to come into close contact with or to be disposed proximately to the surface of the cell culture container base opposite the surface on which the coating is formed, and the coating may be exposed through the mask to light. No particular limitation is imposed on the method for providing a mask so as to come into close contact with or to be disposed proximately to the coating, so long as no damage is caused to the mask or the coating.

No particular limitation is imposed on the light source employed for patternwise light exposure of the coating, so long as the light source can provide light interacting the hydrophilic photosensitive composition. Examples of the light source which may be employed include light sources which emit X-rays, an electron beam, excimer laser beams (e.g., F₂ laser beam, ArF laser beam, and KrF laser beam), a solid-state UV laser beam; metal halide lamps; xenon lamps; and high-pressure mercury lamps. Light exposure energy may be appropriately selected in consideration of the structure of a photosensitive functional group or energy of the light source employed. Generally, light exposure energy is preferably 0.1 mJ/cm² to 5,000 mJ/cm², particularly preferably about 1 mJ/cm² to about 1,000 mJ/cm².

If necessary, thermal treatment may be carried out after light exposure. Conditions for the thermal treatment may be the same as those for thermal treatment which is appropriately performed after application of the photosensitive composition and before light exposure.

No particular limitation is imposed on the method for removing an uncured portion of the exposed coating through development by use of a developer, to thereby form a patterned photo-cured product, so long as the method can dissolve the uncured portion. Examples of preferred methods include a method in which the entirety of the cell culture container base exposed to light is immersed in a developer; and a method in which a developer is applied or sprayed onto the cell culture container base. For example, according to the method in which the cell culture container exposed to light is immersed in a developer, a good patterned photo-cured product can be obtained through immersion in a developer bath for one minute. After formation of a patterned photo-cured product through development, if necessary, the product may be subjected to rinsed.

No particular limitation is imposed on the developer employed for development, so long as the developer exhibits sufficiently different dissolution capabilities between an uncured portion and a cured portion, and does not exhibits adverse effects (e.g., alteration) on the cell culture container. Examples of employable solvents which can dissolve an uncured portion of the coating formed from the photosensitive composition include water, an organic solvent compatible with water, and a mixture thereof. Non-limitative examples of the organic solvent compatible with water include alcohols (e.g., ethanol) and dimethyl sulfoxide. Of these solvents, water is particularly preferred. This is because, similar to the solvent employed for the hydrophilic photosensitive composition, remaining of an organic solvent, which may adversely affect cells, can be avoided by water. Through employment of such a solvent, a pattern with no development residue can be formed. The developer may be a mixture of water and an organic solvent as described above. No particular limitation is imposed on the organic solvent concentration of the developer, so long as the developer can dissolve an uncured portion of the coating. For example, when the developer is a water-ethanol mixture, the ethanol content of the mixture may be a predetermined level within a range of higher than 0 wt.% to lower than 100 wt.%.

No particular limitation is imposed on the drying step performed after development, so long as the developer employed can be removed. The drying step may employ, for example, a thermostat dryer, a hot plate, or an air dryer. Preferably, the drying step is carried out by means of a thermostat dryer at a predetermined temperature. The drying step is generally performed at 30 to 70°C for about 1 minute to about 24 hours, preferably at 30 to 40°C for about 3 minutes to about 1 hour.

Thus, the aforementioned simple method can produce a cell culture container in which each well has, on the bottom surface thereof, a patterned hydrophilic coating layer formed of a photo-cured product of the photosensitive composition. The cell culture container produced through the production method of the present invention can be employed, as is, in biochemical experiments, clinical experiments, and research and development of drugs. The cell culture container does not require a process generally carried out for a plate substrate for culturing cells having a patterned layer on a surface thereof. Specifically, there can be omitted a process in which the substrate is bonded to the surface of a dish (i.e., a type of culture container), and subsequently an aqueous solution containing cells is added to the dish, or a process in which the substrate is placed in a culture dish containing an aqueous solution containing cells. Therefore, the cell culture container is irrelevant to a problem in that a compound eluted from an adhesive employed for such bonding adversely affects cells, or a problem in that cells are deposited not only on the patterned-layer-formed surface (top surface) of the substrate, but also on the bottom surface of the substrate.

The hydrophilic coating layer formed on the bottom surface of each well can reliably maintain the structure thereof in a dry state or in a solution. The hydrophilic coating layer can satisfactorily maintain the structure thereof both in a dry state and a humidified state, and can consistently maintain the structure thereof at about 37°C in water or in an aqueous solvent for a long period of time (e.g., 1 day or longer or 10 days or longer). It is important for the hydrophilic coating layer to be stable in a solution, particularly in water or an organic solvent compatible with water. This is because, since the bottom surface of each well of the cell culture container may be placed in a dry state or exposed to an aqueous solution or an organic solution, the hydrophilic coating layer must be resistant to any of the above conditions. No particular limitation is imposed on the aqueous solvent, so long as the solvent is a solution containing water. Examples of the aqueous solvent include a mixture of water and an organic solvent compatible with water (e.g., an alcohol such as ethanol, or dimethyl sulfoxide); buffers such as aqueous potassium dihydrogenphosphate-disodium hydrogenphosphate solution and aqueous sodium hydrogencarbonate-sodium carbonate solution; aqueous solutions of inorganic and organic salts such as sodium chloride, potassium chloride, and ammonium chloride; aqueous saccharide solutions containing monosaccharide or polysaccharide such as glucose, galactose, glucose, starch, heparin, or heparan sulfate; aqueous protein solutions, aqueous DNA or RNA solutions, liquid culture media, and mixtures thereof. The aqueous solvent may further contain a material which is not dissolved but dispersed in water or the aqueous solvent. Examples of the material include minerals such as clay, fine metal particles such as gold nanoparticles, fine polymer particles such as polystyrene beads and latex particles, animal cells, plant cells, microorganisms, viruses, and mixtures thereof. No particular limitation is imposed on the temperature at which the cell culture container produced through the method of the present invention can be employed, so long as the cell culture container is not adversely affected (e.g., altered or deformed). Generally, the cell culture container is preferably employed at -80°C to 70°C, particularly preferably at 20 to 40°C. This is because, when the cell culture container is employed at a temperature higher than 70°C, photosensitive groups or other groups of the photosensitive resin are decomposed, whereby the photo-cured product may fail to be stably present.

In the cell culture container produced through the production method of the present invention, each well has, on the bottom surface thereof, a portion on which a hydrophilic coating layer is provided (i.e., a non-cell-adhering portion) and a portion where the bottom surface is exposed (i.e., a cell-adhering portion). Therefore, the cell culture container is suitable for use in a new culture system (e.g., cell culturing performed in a specifically patterned area). Specifically, a liquid culture medium containing suspended cells is added to a well of the cell culture container produced through the method of the present invention, cells can be caused to adhere to a portion of the bottom surface of the well other than a portion having thereon a patterned hydrophilic coating layer. Therefore, cells can be cultured in a pattern on the well bottom surface. The hydrophilic coating layer may have a pattern of interest (e.g., a hole pattern, a dot pattern, or a stripe pattern), which is readily formed through appropriately selecting the pattern of the mask employed. The cell culture container of the present invention has a plurality of wells, and thus, if necessary, different types of cells can be cultured in different wells. Therefore, the cell culture container is advantageously employed for various types of evaluation and research/development in relation to cell culture. In addition, since the cell culture container can be produced by forming a pattern on a multi-well plate which is generally used for cell culture, the cell culture container is advantageous in that it can be applied to a conventionally generally used apparatus for multi-well plates; for example, a fluorescence meter such as an immunoreader, or an automatic medium exchanger for treating numerous multi-well plates at one time.

Next will be described, with reference to Figs. 1 to 5, a specific embodiment of the method for producing a cell culture container of the present invention. Fig. 1(a) is a top plan view of a cell culture container base. Fig. 1(b) is a cross-sectional view of the cell culture container base shown in Fig. 1(a), as taken along the line indicated by arrows A and A'. Fig. 2(a) is a top plan view of the cell culture container base in which each well has, on the bottom surface thereof, a coating formed from a hydrophilic photosensitive composition. Fig. 2(b) is a cross-sectional view of the cell culture container base shown in Fig. 2(a), as taken along the line indicated by arrows A and A'. Fig. 3 is a cross-sectional view of a mask. Fig. 4 is a cross-sectional view of the state where the mask is provided on the cell culture container base having thereon coatings formed from the hydrophilic photosensitive composition. Fig. 5(a) is a top plan view of the thus-produced cell culture container. Fig. 5(b) is a cross-sectional view of the cell culture container shown in Fig. 5(a), as taken along the line indicated by arrows A and A'.

As shown in Fig. 1, a cell culture container base 1 has six wells 2. As shown in Fig. 2, a water-soluble photosensitive composition is applied to the bottom surfaces of the wells 2 of the cell culture container base 1, to thereby form coatings 3. Subsequently, the coatings 3 are patternwise exposed to light through a mask 10 shown in Fig. 3. A mask base 11 is made of a transparent material. As shown in Fig. 3, the mask base 11 has cylindrical protrusions 12 which are inserted into the wells 2, and each of the cylindrical protrusions 12 has, at a tip end 13 thereof which faces the bottom surface of a corresponding well 2; i.e., faces the coating 3 formed on the bottom surface, a pattern 14 formed through vapor deposition of chromium and having 16 non-translucent dots. As shown in Fig. 4, the mask 10 is provided on the cell culture container base 1 so that the tip ends 13 are inserted into the wells 2, and the tip ends 13 come into close contact with or are disposed proximately to the coatings 3. Subsequently, the coatings 3 are patternwise exposed to light; i.e., the coatings 3 are exposed to light through the mask 10. The thus-exposed portions are cured through photo-crosslinking, and the non-exposed portions are removed through development. Thus, as shown in Fig. 5, a coating layer formed of a photo-cured product of the hydrophilic photosensitive composition and having a pattern 5 (i.e., a pattern of dot-like holes 4) is provided on the bottom surface of each of the wells 2 (the coating layer corresponds to a "patterned hydrophilic coating layer formed of a photo-cured product" as described in claims).

### Examples

The present invention will next be described by way of Examples, which should not be construed as limiting the invention thereto.

### (Synthesis Example 1) Synthesis of photosensitive resin A

Polyethylene glycol-diamine (product of NOF Corporation, number average molecular weight of 1,000) (7.9 g), photo-functional compound 4 (2-(2-(4-azidophenyl)vinyl)-4-(3-pyridylmethylene)-1,3-oxazolin-5-one) (10.0 g, which is 2.0 equivalents by mole of amino groups of polyethylene glycol-diamine), and tetrahydrofuran (THF) (70 g) were mixed, and the mixture was allowed to react at 25°C for 18 hours. After completion of reaction, THF was removed through evaporation. Subsequently, the reaction mixture was subjected to partition and extraction with water (50 g) and ethyl acetate (50 g). After removal of the organic layer, another aliquot (50 g) of ethyl acetate was added, and partition-extraction was repeated. After the mixture had been allowed to stand still, the mixture was separated into three phases. The thus-obtained lowest oil phase was lyophilized, to thereby yield 7.2 g of photosensitive resin A represented by the following formula (a) (n = 23). The thus-produced photosensitive resin A was identified as a target compound through ¹H-NMR analysis on the basis of a proton peak attributed to methylene chains of polyethylene oxide (3.5 ppm) and proton peaks attributed to aromatic rings of photo-functional compound 4 (6.8 ppm to 8.7 ppm). The percent introduction of photo-functional compound 4, as calculated from integral peak intensity ratio, was 95%.

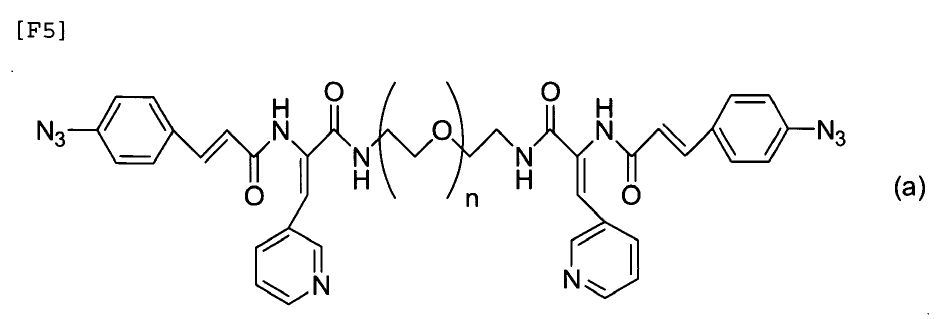

### (Synthesis Example 2) Synthesis of photosensitive resin B

The procedure of Synthesis Example 1 was repeated, except that polyethylene glycol-diamine (product of NOF Corporation, number average molecular weight of 2,000) (7.9 g) and photo-functional compound 4 (2-(2-(4-azidophenyl)vinyl)-4-(3-pyridylmethylene)-1,3-oxazolin-5-one) (6.0 g, which is 2.4 equivalents by mole of amino groups of polyethylene glycol-diamine) were employed. In partition-extraction, the aqueous phase was washed twice with an organic phase, and the washed aqueous phase was lyophilized, to thereby yield 7.0 g of photosensitive resin B represented by formula (a) (n = 45). The thus-produced photosensitive resin B was identified as a target compound through ¹H-NMR analysis on the basis of a proton peak attributed to methylene chains of polyethylene oxide (3.5 ppm) and proton peaks attributed to aromatic rings of photo-functional compound 4 (6.8 ppm to 8.7 ppm). The percent introduction of photo-functional compound 4, as calculated from integral peak intensity ratio, was 96%.

### (Synthesis Example 3) Synthesis of photosensitive resin C

The procedure of Synthesis Example 1 was repeated, except that polyethylene glycol-dipropylamine (product of Wako Pure Chemical Industries, Ltd., number average molecular weight of 9,000 to 10,000) (23.7 g), photo-functional compound 4 (2-(2-(4-azidophenyl)vinyl)-4-(3-pyridylmethylene)-1,3-oxazolin-5-one) (4.0 g, which is 2.4 equivalents by mole of amino groups of polyethylene glycol-dipropylamine), tetrahydrofuran (65 g), and acetonitrile (65 g) were mixed, to thereby yield 23.2 g of photosensitive resin C represented by the following formula (b) (n = 216). The thus-produced photosensitive resin C was identified as a target compound through ¹H-NMR analysis on the basis of a proton peak attributed to methylene chains of polyethylene oxide (3.5 ppm) and proton peaks attributed to aromatic rings of photo-functional compound 4 (6.8 ppm to 8.7 ppm). The percent introduction of photo-functional compound 4, as calculated from integral peak intensity ratio, was 90%.

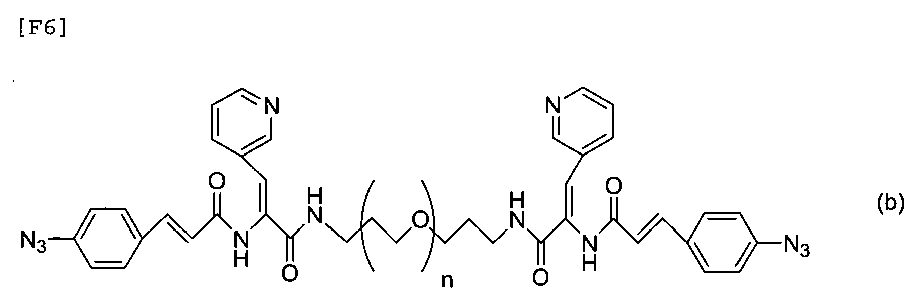

### (Synthesis Example 4) Synthesis of photosensitive resin D

The procedure of Synthesis Example 1 was repeated, except that polyethylene glycol-diamine (product of NOF Corporation, number average molecular weight of 2,000) (17.2 g) and photo-functional compound 3 (2-(4-azidophenyl)-4-(3-pyridylmethylene)-1,3-oxazolin-5-one) (6.0 g, which is 1.2 equivalents by mole of amino groups of polyethylene glycol-diamine) were employed, to thereby yield 19.7 g of photosensitive resin D represented by the following formula (c) (n = 45). The thus-produced photosensitive resin D was identified as a target compound through ¹H-NMR analysis on the basis of a proton peak attributed to methylene chains of polyethylene oxide (3.5 ppm) and proton peaks attributed to aromatic rings of photo-functional compound 3 (6.8 ppm to 8.7 ppm). The percent introduction of photo-functional compound 3, as calculated from integral peak intensity ratio, was 70%.

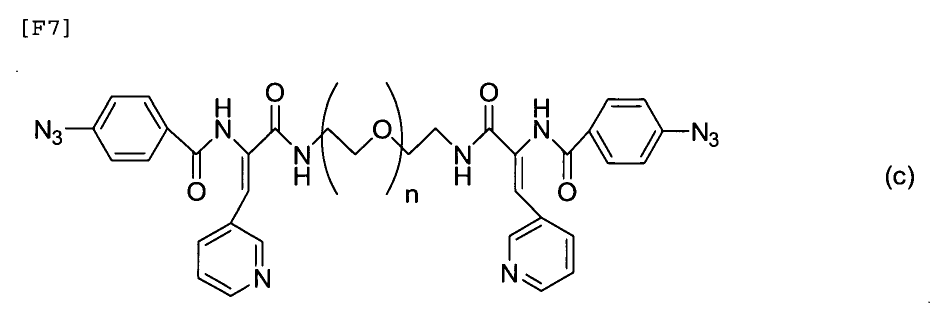

### (Synthesis Example 5) Synthesis of photosensitive resin E

The procedure of Synthesis Example 1 was repeated, except that polyethylene glycol-diamine (product of NOF Corporation, molecular weight of 2,000) (15.1 g) and photo-functional compound 6 (4-(4-azido-β-methyl-cinnamylidene)-2-(3-pyridyl)-2-oxazolin-5-one) (6.0 g, which is 1.2 equivalents by mole of amino groups of polyethylene glycol-diamine) were employed, to thereby yield 18.4 g of photosensitive resin E represented by the following formula (d) (n = 45). The thus-produced photosensitive resin E was identified as a target compound through ¹H-NMR analysis on the basis of a proton peak attributed to methylene chains of polyethylene oxide (3.5 ppm) and proton peaks attributed to aromatic rings of photo-functional compound 6 (6.8 ppm to 8.7 ppm). The percent introduction of photo-functional compound 6, as calculated from integral peak intensity ratio, was 71%.

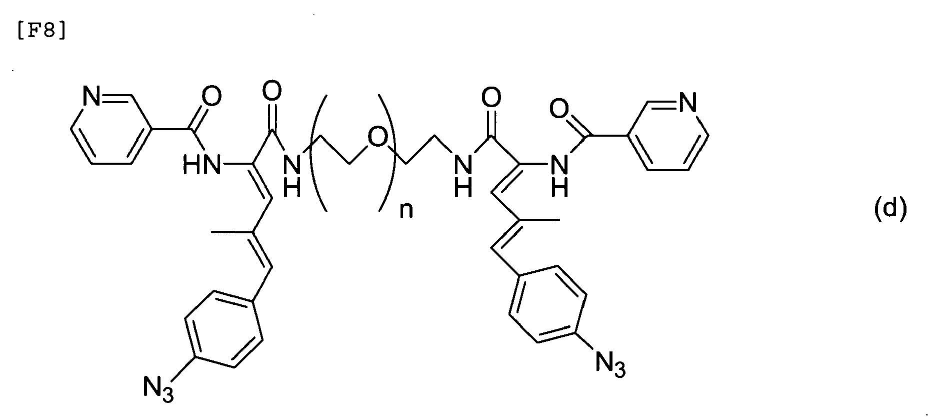

### (Synthesis Example 6) Synthesis of photosensitive resin F

Polyvinyl alcohol (EG-30, product of Nippon Synthetic Chemical Industry Co., Ltd.) (50 g) was dissolved in water (450 g). To the resultant solution were added a photo-functional compound (2-(3-(4-azidophenyl)prop-2-enoylamino)-N-(4,4'-dimethoxybutyl)-3-(3-pyridyl)prop-2-eneamide) (3.5 g) synthesized according to Synthesis Example 5 described in Japanese Patent Application Laid-Open (*kokai*) No. 2003-292477 and phosphoric acid (1.5 g), followed by reaction at 60°C for 24 hours. The percent acetalization was found to be 97%. Phosphoric acid was removed through an ion-exchange treatment, to thereby prepare photosensitive resin F (percent introduction of photosensitive groups: 0.8 mol% with respect to hydroxyl groups of PVA).

### (Synthesis Example 7) Synthesis of photosensitive resin G

Polyvinyl alcohol (EG-30, product of Nippon Synthetic Chemical Industry Co., Ltd.) (100 g) was dissolved in water (700 g) and methanol (200 g). To the resultant solution were added a photo-functional compound (3-(4-azidophenyl)-N-(4,4'-dimethoxybutyl)-2-phenylcarbonylaminoprop-2-eneamide) (10 g) synthesized according to Synthesis Example 1 described in Japanese Patent Application Laid-Open (*kokai*) No. 2003-292477 and phosphoric acid (3 g), followed by reaction at 60°C for 24 hours. The percent acetalization was found to be 97%. Phosphoric acid was removed through an ion-exchange treatment, to thereby prepare photosensitive resin G (percent introduction of photosensitive groups: 0.8 mol% with respect to hydroxyl groups of PVA).

### (Synthesis Example 8) Synthesis of photosensitive resin H

Polyvinyl alcohol (EG-30, product of Nippon Synthetic Chemical Industry Co., Ltd.) (100 g) was dissolved in water (900 g). To the resultant solution were added a photo-functional compound (3-(4-azidophenyl)-N-(4,4'-dimethoxybutyl)-2-[(3-pyridyl)carbonylamino]-prop-2-eneamide) (10 g) synthesized according to Synthesis Example 3 described in Japanese Patent Application Laid-Open (*kokai*) No. 2003-292477 and phosphoric acid (3 g), followed by reaction at 60°C for 24 hours. The percent acetalization was found to be 97%. Phosphoric acid was removed through an ion-exchange treatment, to thereby prepare photosensitive resin H (percent introduction of photosensitive groups: 0.8 mol% with respect to hydroxyl groups of PVA).

### (Synthesis Example 9) Synthesis of photosensitive resin I

Amino-group-introduced polyethylene glycol having repeating units represented by the following formula (e) and having hydroxyl groups at both backbone ends (product of NOF Corporation, molecular weight of 3,200, average repeating unit number: x = 3.1, y = 60.0) (0.5 g), the aforementioned photo-functional compound 4 (2-(2-(4-azidophenyl)vinyl)-4-(3-pyridylmethylene)-1,3-oxazolin-5-one) (0.2 g, which is 1.5 equivalents by mole of amino groups of the polyethylene glycol derivative), and tetrahydrofuran (THF) (7 g) were mixed, and the mixture was allowed to react at 25°C for 19 hours. After completion of reaction, THF was removed under reduced pressure. Subsequently, the reaction mixture was subjected to partition and extraction with water (7 g) and ethyl acetate (7 g). After removal of the organic layer, another aliquot (7 g) of ethyl acetate was added, and partition-extraction was repeated. After the mixture had been allowed to stand still, the aqueous phase was separated. The aqueous phase was lyophilized, to thereby yield 0.6 g of photosensitive resin I having repeating units represented by the following formula (f) and having hydroxyl groups at both backbone ends (average repeating unit number: m = 3.0, n = 60.0, p = 0.1). The thus-produced photosensitive resin I was identified as a target compound through ¹H-NMR analysis on the basis of a proton peak attributed to methylene chains of polyethylene oxide (3.5 ppm) and proton peaks attributed to aromatic rings of photo-functional compound 4 (6.8 ppm to 8.7 ppm). The ratio m/(m + n), as calculated from integral peak intensity ratio, was 0.048.

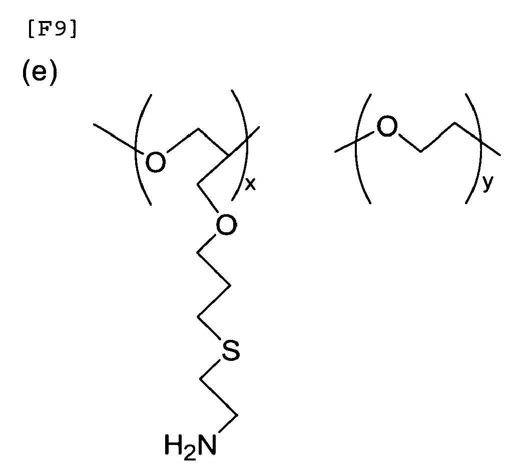

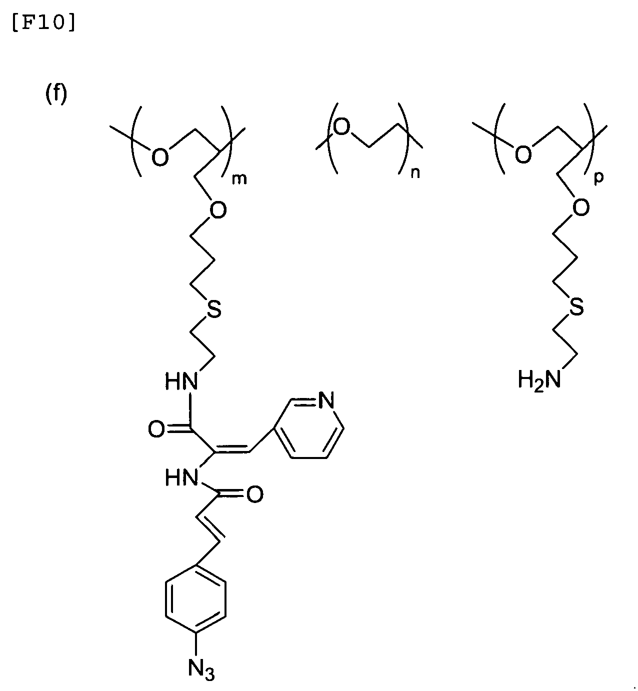

### (Preparation of photosensitive composition I)

Photosensitive resin A produced in Synthesis Example 1 was mixed with an aqueous medium whose pH was adjusted to 3 with hydrochloric acid so as to attain a total solid content (wt.%) shown in Table 2. The thus-prepared aqueous solution was filtered through a 0.45-µm cellulose acetate membrane filter (hereinafter referred to simply as a "filter"), to thereby yield photosensitive composition I-1.

### (Preparation of photosensitive compositions II (II-1 to II-4) to IX)

The procedure of preparation of photosensitive composition I was repeated, except that photosensitive resin A was substituted by a photosensitive resin as shown in Table 2 or 3; the total solid content (wt.%) was determined as shown in Table 2 or 3; and pure water was employed in place of the aqueous medium having a pH of 3, to thereby yield photosensitive compositions (II to IX).

### (Example 1)

There was employed a polystyrene flat-bottom 96-well plate (trademark "Sumilon Mutiplate 96F," product of Sumitomo Bakelite Co., Ltd., 0.32 cm²/well, hereinafter referred to as a "non-coated resin plate"), which is a general-purpose multi-well plate. The above-prepared photosensitive composition I-1 was dispensed into the wells of the 96-well plate by means of a pipette so that the amount of the composition applied to each well was 10 µL. Thereafter, the 96-well plate was allowed to stand still in a thermostat dryer at 60°C for 30 minutes, to thereby form a coating of photosensitive composition I-1 on each well.

On the coating formed on each well was placed a quartz mask of 4 mm x 4 mm having a dot pattern (having 169 dots (150 µmφ each) formed through vapor deposition of chromium) by means of adsorption tweezers, and the coating was exposed, through the mask, to light from a high-pressure mercury lamp (1,000 mJ/cm²). Thus, only an exposed portion of the coating was photo-cured. Thereafter, the multi-well plate was immersed in a water bath for development at 25°C for one minute, followed by drying at 60°C for 10 minutes, to thereby yield a cell culture container formed of the 96-well plate having, on the bottom surface of each well, a photo-cured product of photosensitive composition I-1 having dot-like holes corresponding to the aforementioned dot pattern. The above-described procedure was repeated, except that the amount of the photosensitive composition applied to each well was changed to 50 µL and 100 µL, to thereby yield other cell culture containers.

### (Examples 2 to 9)

The procedure of Example 1 was repeated, except that photosensitive resin I-1 was substituted by each of the photosensitive resins as shown in Table 2 and 3, and the amount of the photosensitive resin applied to each well, and light exposure dose were determined as shown in Table 2 or 3, to thereby yield a cell culture container formed of the 96-multi-well plate having, on the bottom surface of each well, a photo-cured product of the photosensitive composition having dot-like holes corresponding to the aforementioned dot pattern. For production of a cell culture container by use of each of photosensitive compositions II-1 to II-4 and VI-1 to VI-4, drying of a coating formed from the photosensitive composition was also carried out under the conditions of 60°C x one hour, 60°C x 15 hours, 37°C x 30 minutes, and 37°C x one hour. For production of a cell culture container by use of each of photosensitive compositions II-1 to II-4 and VI-1 to VI-4, there was also employed another general-purpose multi-well plate, which is a type I collagen-coated polystyrene flat-bottom 96-well plate (trademark "Sumilon Celltight C-1 Plate 96F," product of Sumitomo Bakelite Co., Ltd., 0.32 cm²/well, hereinafter referred to as a "collagen-coated plate"); a glass flat-bottom 96-well plate (product of Nippon Sheet Glass Co., Ltd., hereinafter referred to as a "non-coated glass plate"); or an aminosilane-coated polystyrene flat-bottom 96-well plate having a surface provided with amino groups (trademark "Sumilon Celltight PL Plate 96F," product of Sumitomo Bakelite Co., Ltd., 0.32 cm²/well, hereinafter referred to as an "APS-coated resin plate"). The aforementioned drying conditions and conditions shown in Table 3 were employed.

**[Table 2]**

| | Composition | Photosensitive resin | | Application amount (µL) | | | Light exposure dose mJ/cm² |
|---|---|---|---|---|---|---|---|
| | | Type | wt.% | | | | |
| Ex. 1 | I-1 | Photosensitive resin A | 2.5 | 100 | 50 | 10 | 1,000 |
| Ex. 3 | III-1 | Photosensitive resin C | 2.5 | 100 | 50 | 10 | 1,000 |
| Ex. 4 | IV-1 | Photosensitive resin D | 1.0 | 100 | 50 | 10 | 1,000 |
| Ex. 5 | V-1 | Photosensitive resin E | 2.5 | 100 | 50 | 10 | 1,000 |
| Ex. 7 | VII-1 | Photosensitive resin G | 1.0 | 100 | 50 | 10 | 15 |
| Ex. 8 | VIII-1 | Photosensitive resin H | 0.5 | 100 | 50 | 10 | 15 |
| Ex. 9 | IX-1 | Photosensitive resin I | 1.0 | 100 | 50 | 10 | 100 |

**[Table 3]**

| | Composition | Photosensitive resin | | Application amount (µL) | | | | | Light exposure dose mJ/cm² |
|---|---|---|---|---|---|---|---|---|---|
| | | Type | wt.% | | | | | | |
| Ex. 2 | II-1 | Photosensitive resin B | 2.5 | 100 | 50 | 15 | 10 | 5 | 1,000 |
| | II-2 | Photosensitive resin B | 1.0 | 100 | 50 | 15 | 10 | 5 | 1,000 |
| | II-3 | Photosensitive resin B | 0.5 | 100 | 50 | 15 | 10 | 5 | 1,000 |
| | II-4 | Photosensitive resin B | 0.2 | 100 | 50 | 15 | 10 | 5 | 1,000 |
| Ex. 6 | VI-1 | Photosensitive resin F | 2.5 | 100 | 50 | 15 | 10 | 5 | 15 |
| | VI-2 | Photosensitive resin F | 1.0 | 100 | 50 | 15 | 10 | 5 | 15 |
| | VI-3 | Photosensitive resin F | 0.5 | 100 | 50 | 15 | 10 | 5 | 15 |
| | VI-4 | Photosensitive resin F | 0.2 | 100 | 50 | 15 | 10 | 5 | 15 |

### (Example 10)

There was employed a polystyrene flat-bottom 12-multi-well plate (trademark "Sumilon Mutiplate 12F," product of Sumitomo Bakelite Co., Ltd., 3.6 cm²/well), which is a general-purpose multi-well plate. Photosensitive composition II-1 prepared in Example 2 was dispensed into the wells of the 12-well plate so that the amount of the composition applied to each well was 100 µL. Thereafter, the 12-well plate was allowed to stand still in a thermostat dryer at 60°C for 30 minutes, to thereby form a coating of photosensitive composition II-1 on each well.

On the coating formed on each well was placed a quartz mask of 10 mm x 10 mm having a dot pattern (having 900 dots (150 µmφ each) formed through vapor deposition of chromium) by means of adsorption tweezers, and the coating was exposed, through the mask, to light from a high-pressure mercury lamp (1,000 mJ/cm²). Thus, only an exposed portion of the coating was photo-cured. Thereafter, the multi-well plate was immersed in a water bath for development at 25°C for one minute, followed by drying at 60°C for 10 minutes, to thereby yield a cell culture container formed of the 96-multi-well plate having, on the bottom surface of each well, a photo-cured product of photosensitive composition II-1 having dot-like holes corresponding to the aforementioned dot pattern.

### (Example 11)

The procedure of Example 1 was repeated, except that the mask of 4 mm x 4 mm having the 150-µmφ dot pattern was substituted by a mask shown in Fig. 6, and photosensitive resin I-1 was substituted by photosensitive resin II-1, II-2, II-3, or II-4, to thereby yield a cell culture container formed of the 96-multi-well plate having, on the bottom surface of each well, a photo-cured product of photosensitive composition II-1, II-2, II-3, or II-4 having dot-like holes corresponding to the dot pattern. Fig. 6(a) is a top plan view of the mask employed in Example 11; Fig. 6(b) is an enlarged view of a portion of the tip end of a protrusion of the mask shown in Fig. 6(a); and Fig. 6(c) is a cross-sectional view of the mask shown in Fig. 6(a), as taken along the line indicated by arrows A and A'. As shown in Fig. 6, the mask 20 is formed of a quartz plate substrate 21 (115 mm x 75 mm) having thereon 96 quartz cylindrical columns 22 (6 mmφ, height of 11.6 mm each) which are arranged so as to correspond to the wells of the multi-well plate. Each of the cylindrical columns 22 has, on the tip end (top) 23 thereof, a pattern of 135 dots 24 (100 µmφ each) formed through vapor deposition of chromium.

### (Example 12)

The procedure of Example 1 was repeated, except that the mask of 4 mm x 4 mm having the 150-µmφ dot pattern was substituted by a mask formed of a quartz plate substrate (115 mm x 75 mm) having thereon a pattern of 169 dots (100 µmφ each) formed through vapor deposition of chromium; the mask was brought into close contact with the bottom surface of the 96-multi-well plate; and a coating formed on each well was exposed to light through the plate bottom surface, to thereby yield a cell culture container formed of the 96-multi-well plate having, on the bottom surface of each well, a photo-cured product of photosensitive composition II-1, II-2, II-3, or II-4 having dot-like holes corresponding to the dot pattern.

### (Test Example) Solvent exposure test

In each of the cell culture containers produced in Examples 1 to 12, an aqueous solvent of 25°C or 37°C was added to each well. Three days and 21 days after addition of the solvent, the hydrophilic coating layer on the bottom surface of each well was observed, to thereby evaluate the shape of the hydrophilic coating layer, and to determine whether or not the layer was exfoliated from the well bottom surface. The aqueous solvent employed was pure water or an aqueous potassium dihydrogenphosphate-disodium hydrogenphosphate solution (phosphate buffer, pH: 7.4). Figs. 7(a) to 7(d) show, as examples, the states of hydrophilic coating layers which had been immersed in phosphate buffer for 21 days. Specifically, Fig. 7(a) shows the post-immersion state of a hydrophilic coating layer formed by applying photosensitive composition II-1 to a collagen-coated plate (5 µL for each well), followed by drying at 60°C for 30 minutes; Fig. 7(b) shows the post-immersion state of a hydrophilic coating layer formed by applying photosensitive composition II-1 to a collagen-coated plate (5 µL for each well), followed by drying at 37°C for 30 minutes; Fig. 7(c) shows the post-immersion state of a hydrophilic coating layer formed by applying photosensitive composition VI-1 to a collagen-coated plate (10 µL for each well), followed by drying at 60°C for 30 minutes; and Fig. 7(d) shows the post-immersion state of a hydrophilic coating layer formed by applying photosensitive composition VI-1 to a collagen-coated plate (10 µL for each well), followed by drying at 60°C for 15 hours.

The hydrophilic coating layer formed on the bottom surface of each well was stable in terms of surface morphology before and after immersion in the solvent. Specifically, the hydrophilic coating layer exhibited no change in surface conditions. In addition, exfoliation or breakage of the coating layer, which would otherwise be caused by swelling, did not occur. Therefore, the hydrophilic coating layer formed on the bottom surface of each well of the cell culture container produced through the production method of the present invention was found to exhibit resistance to an aqueous solvent, and to have an ability to maintain its performance in short-term to long-term culturing.

## Claims

1. A method for producing a cell culture container, **characterized by** comprising providing a cell culture container base having a plurality of wells serving as regions for culturing cells; applying a hydrophilic photosensitive composition to the bottom surface of each of the wells, to thereby form a coating; subjecting the coating to patternwise light exposure; and removing an uncured portion of the coating through development, to thereby yield a cell culture container having, on the bottom surface of each well, a patterned hydrophilic coating layer formed of a photo-cured product.

2. A method for producing a cell culture container as described in claim 1, wherein the hydrophilic photosensitive composition contains a photosensitive resin having a water-soluble polymer backbone and a photo-crosslinkable photosensitive group.

3. A method for producing a cell culture container as described in claim 1 or 2, wherein the patternwise light exposure is carried out using a mask having protrusions, each protrusion having a pattern of interest on the tip end thereof which faces the bottom surface of a corresponding well of the cell culture container and being inserted in the well such that the tip end of the protrusion comes into close contact with or is disposed proximately to the coating formed on the bottom surface of the well.
